(19) Europäisches Patentamt
European Patent Office
Office européen des brevets

(11) Veröffentlichungsnummer: **0 544 118 B1**

(12) **EUROPÄISCHE PATENTSCHRIFT**

(45) Veröffentlichungstag der Patentschrift: **09.08.95**

(51) Int. Cl.[6]: **C07C 45/64**, C07C 47/19, C07C 31/20, C07C 29/141

(21) Anmeldenummer: **92118470.1**

(22) Anmeldetag: **29.10.92**

# (54) Verfahren zur Herstellung von 3-Hydroxyalkanalen.

(30) Priorität: **27.11.91 DE 4138981**

(43) Veröffentlichungstag der Anmeldung:
**02.06.93 Patentblatt 93/22**

(45) Bekanntmachung des Hinweises auf die Patenterteilung:
**09.08.95 Patentblatt 95/32**

(84) Benannte Vertragsstaaten:
**AT BE CH DE DK ES FR GB IT LI LU NL PT SE**

(56) Entgegenhaltungen:
**DE-C- 922 166**

(73) Patentinhaber: **Degussa Aktiengesellschaft**
**Weissfrauenstrasse 9**
**D-60311 Frankfurt (DE)**

(72) Erfinder: **Haas, Thomas, Dr.**
**Hölderlinstrasse 20**
**W-6000 Frankfurt am Main 1 (DE)**
Erfinder: **Böhme, Georg**
**Nordring 49**
**W-6458 Rodenbach (DE)**
Erfinder: **Arntz, Dietrich, Dr.**
**Lorsbachstrasse 32**
**W-6370 Oberursel (DE)**

EP 0 544 118 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung richtet sich auf ein Verfahren zur Herstellung von 3-Hydroxyalkanalen mit 3 bis 12, insbesondere 3 oder 4, Kohlenstoffatomen durch Hydratisierung der zugrundeliegenden 2-Alkenale mit Wasser in homogener Phase in Gegenwart eines sauren Katalysators.

2-Alkenale der allgemeinen Formel $H_2C=CR\text{-}CHO$ mit R gleich Wasserstoff oder Alkyl, insbesondere Acrolein und Methacrolein, lassen sich gemäß US 2,434,110 in Gegenwart saurer Katalysatoren mit Wasser hydratisieren, wobei die entsprechenden 3-Hydroxyalkanale gebildet werden. Aus Acrolein ist 3-Hydroxypropionaldehyd (HPA) zugänglich, woraus durch Hydrierung das als Monomerbaustein für Polyester und Polyurethane zunehmend Bedeutung erlangende 1,3-Propandiol herstellbar ist.

Im Verfahren gemäß der US 2,434,110 werden als Katalysator im Reaktionsgemisch homogen gelöste Säuren, wie beispielsweise Schwefelsäure, Salzsäure, Phosphorsäure, Oxalsäure, saure Salze, Essigsäure, verwendet; als bevorzugt wird Schwefelsäure herausgestellt. Nachteilig an diesem Verfahren sind die niedrigen Ausbeuten und niedrigen Selektivitäten.

Um die Selektivität der Hydratisierung von Acrolein zu verbessern, wurden weitere Verfahren entwickelt. Unter Verwendung von Kohlendioxid als Katalysator - siehe GB 1,185,615 - sind zwar brauchbare Selektivitäten erzielbar, aber die erforderliche lange Reaktionszeit mindert in erheblichem Umfang die Raum-Zeit-Ausbeute des Verfahrens. Schließlich können auch heterogene Katalysatoren, nämlich schwach saure, carboxylgruppenhaltige Ionenaustauscher eingesetzt werden - siehe US 3,536,763. In der Praxis zeigte sich, daß konventionelle carboxylgruppenhaltige Ionenaustauscher eine begrenzte Wirksamkeit zeigen und damit lange Reaktionszeiten erfordern. Eine Verbesserung bezüglich der Raum-Zeit-Ausbeute bei gleichzeitig hoher Selektivität erbrachten Ionenaustauscher mit Phosphonsäuregruppen - siehe DE-OS 39 26 136.

Als Alternative zu den heterogenen Katalysatoren besteht weiterhin ein Interesse daran, in homogener Phase wirksame Katalysatorsysteme zu finden, welche eine Hydratisierung der 2-Alkenale mit hoher Selektivität bei gleichzeitig befriedigender Raum-Zeit-Ausbeute erlauben. Bevorzugte Katalysatorsysteme sollten zudem in einfacher Weise im Rahmen der Aufarbeitung eines Folgeprodukts der 3-Hydroxyalkanale, wie insbesondere der daraus durch Hydrierung erhältlichen Alkan-1,3-diole rückgewinnbar sein. Üblicherweise wird das Reaktionsgemisch aus der Hydratisierungsstufe nur von nicht umgesetztem 2-Alkenal befreit und das 3-Hydroxyalkanal ohne vorherige Isolierung in ein Folgeprodukt überführt.

Gefunden wurde ein Verfahren zur Herstellung von 3-Hydroxyalkanalen mit 3 bis 12 C-Atomen durch Hydratisierung der zugrundeliegenden 2-Alkenale mit Wasser in homogener Phase in Gegenwart eines sauren Katalysatores bei einer Reaktionstemperatur von 20 °C bis 120 °C, einem Druck von 1 bar bis 20 bar und einer Anfangskonzentration des 2-Alkenals im Reaktionsgemisch von 3 bis 30 Gew.-%, das dadurch gekennzeichnet ist, daß man als Katalysator einen gelösten Säure-Base-Puffer auf der Basis (a) einer einwertigen Carbonsäure und eines löslichen Salzes dieser Säure oder (b) einer mehrwertigen Carbonsäure oder Phosphorsäure und eines Salzes aus diesen Säuren mit tertiären Aminen oder N-Heteroaromaten verwendet, der im Reaktionsgemisch zu einem pH-Wert von 2 bis 5 führt und dessen Säurekomponente und korrespondierende Base im Reaktionsgemisch in einer Menge von insgesamt 0,5 bis 40 Gew.-% anwesend sind.

Bei den 2-Alkenalen handelt es sich vorzugsweise um Acrolein und Methacrolein und ganz besonders um Acrolein.

Säure-Base-Puffer, welche sich im Rahmen der Aufarbeitung eines Folgeprodukts des 3-Hydroxyalkanals abtrennen lassen, basieren auf einer einwertigen Carbonsäure mit einem Siedepunkt unter Normaldruck von unter 200 °C, vorzugsweise unter 160 °C, und eines Salzes derselben mit einem tertiären Amin oder N-Heteroaromat, wobei das tertiäre Amin oder der N-Heteroaromat einen Siedepunkt unter Normaldruck unter 200 °C, vorzugsweise unter 160 °C, aufweisen.

Die zuvor genannten Säure-Base-Puffer führen bei Destillationsprozessen zu keinen Verkrustungen. Vielmehr lassen sie sich destillativ aus Reaktionsgemischen abtrennen, weil zwischen dem Salz und seinen Bestandteilen ein Gleichgewicht besteht:

$$R^1COO^{\ominus}\ H\overset{\oplus}{N}R_3 \rightleftarrows R^1COOH + NR_3 .$$

Bei der Auswahl der Säure und des Amins wird der Fachmann solche Kombinationen wählen, welche sich problemlos thermisch spalten lassen und deren Siedepunkte ausreichend weit von dem gewünschten Folgeprodukt des 3-Hydroxyalkanals sieden, so daß dieses Folgeprodukt in reiner Form gewonnen werden

kann. Besonders bevorzugte Puffersysteme enthalten als Säurekomponente Essigsäure oder Propionsäure und als Amin Trimethylamin, Triethylamin und Tripropylamin oder Pyridin.

Im Falle der Hydratisierung von beispielsweise Acrolein und Hydrierung des entstandenen 3-Hydroxypropionaldehyds zu 1,3-Propandiol kann als Säurekomponente für den Puffer vorzugsweise auch Acrylsäure verwendet werden. Dies hat den Vorteil, daß durch Propenoxidation erhaltenes Rohacrolein, das als Nebenprodukt Acrylsäure enthält, in der Hydratisierungsstufe eingesetzt werden kann. Die Abtrennung der Acrylsäure erfolgt dann, sofern erwünscht, erst nach der Hydratisierung des Acroleins. Es bedarf somit nicht des Einsatzes einer artfremden Säurekomponente.

Unter den für die Herstellung des Puffersystems (a) einsetzbaren Basen sind außer den bereits hervorgehobenen tertiären Aminen und N-Heterocyclen auch alkalisch wirkende Salze der Carbonsäuren, etwa deren Alkali- oder Erdalkalisalze sowie Hydroxide und Carbonate der Alkali- und Erdalkalimetalle zu nennen. Für die Hydratisierung selbst ist es von geringer Bedeutung, ob im Reaktionsgemisch Trialkylammoniumionen, protonierte N-Heterocyclen, Alkali- oder Erdalkaliionen anwesend sind. Im Falle der Anwesenheit von Alkali- und/oder Erdalkaliionen verbleiben aber Salze hiervon nach Abtrennung des 3-Hydroxyalkanals bzw. seines Folgeproduktes aus dem Reaktionsgemisch im Destillationssumpf und bereiten damit Schwierigkeiten beim Entsorgen desselben durch Verbrennung. Prinzipiell können anstelle tertiärer Amine auch sekundäre Amine zur pH-Einstellung des Puffers herangezogen werden, jedoch wird dadurch die Selektivität der Hydratisierung wesentlich gesenkt.

Ein bevorzugter pH-Bereich für die Durchführung der Hydratisierung liegt zwischen 3 und 4,5 , insbesondere zwischen 3 und 4. Hierbei zeigte sich in nicht vorhersehbarer Weise, daß der pH-Wert allein keine ausreichende technische Lehre darstellt, um eine hohe Selektivität bei gleichzeitig hoher Raum-Zeit-Ausbeute zu erzielen. Erst durch die Verwendung der beanspruchten Puffersysteme läßt sich dieses Ziel erreichen. Wird bei vorgegebenem pH-Wert anstelle eines Puffers die ihm zugrundeliegende Säurekomponente allein verwendet, sinkt die Reaktionsgeschwindigkeit auf Werte ab, welche die Wirtschaftlichkeit des Verfahrens völlig infrage stellen.

Die Herstellung des Puffers bzw. Einstellung des pH-Wertes im Reaktionsgemisch erfolgt in üblicher Weise. Vorzugsweise wird zu einer wäßrigen Lösung der ausgewählten Säurekomponente die gewünschte Basekomponente, vorzugsweise also ein tertiäres Amin, bis zum Erreichen des gewünschten pH-Wertes zugeführt.

Das Reaktionsgemisch setzt sich aus einer wäßrigen Pufferlösung und dem zu hydratisierenden 2-Alkenal zusammen. Die Säurekomponente des Puffers und die hierzu korrespondierende Base - also beispielsweise die Acrylsäure und das Acrylatanion - sind im Reaktionsgemisch in einer Menge von insgesamt 0,5 bis 40 Gew.-%, vorzugsweise 0,5 bis 10 Gew.-% und insbesondere 0,5 bis 5 Gew.-%, enthalten. Die Menge an Puffer und 2-Alkenal im Reaktionsgemisch werden so aufeinander abgestimmt, daß das Reaktionsgemisch bei der Reaktionstemperatur eine homogene Phase bildet. Eine bevorzugte Anfangskonzentration an 2-Alkenal im Reaktionsgemisch liegt im Bereich von 6 bis 20 Gew.-%, insbesondere 10 bis 18 Gew.-%. Die Hydratisierung kann in einem weiteren Temperaturbereich erfolgen, bevorzugt wird eine Temperatur von 50 bis 90 °C. Üblicherweise wird unter Normaldruck gearbeitet, bei Temperaturen um/oberhalb des Siedepunktes des eingesetzten 2-Alkenals wird aber zweckmäßigerweise ein geringer Überdruck angewendet.

Wie bereits oben dargelegt, wird das bei der Hydratisierung erhaltene Reaktionsgemisch meist direkt der Folgeproduktherstellung zugeführt. Hierbei wird aus dem Reaktionsgemisch zunächst nicht umgesetztes 2-Alkenal abdestilliert und der Hydratisierungsstufe wieder zugeführt.

Der gesamte Katalysator, Amin und Säure, kann nach der Hydrierung zusammen mit dem Wasser abdestilliert und rezykliert werden. Alternativ kann das im Salz gebundene Amin, falls es einen Siedepunkt von $<$ 100 °C hat, auch direkt nach der Hydratisierung durch Destillation abgetrennt und zurückgeführt werden. Bei Verwendung von Propionsäure als Säurekomponente des Puffers kann diese auch teilweise nach der Hydratisierung als Azeotrop mit $H_2O$ abdestilliert werden.

Vorteile des erfindungsgemäßen Verfahrens sind die sicher erzielbaren hohen Selektivitäten bei guten Raum-Zeit-Ausbeuten, die Einsetzbarkeit von Roh-2-alkenalen, wie z. B. acrylsäurehaltigem Rohacrolein, die einfache Rezyklierbarkeit der Bestandteile des Puffers auf der Basis von Monocarbonsäuren und tertiären Aminen oder N-Heteroaromaten im Rahmen der Aufarbeitung von destillierbaren Folgeprodukten. insbesondere 1,3-Diolen.

Die Erfindung wird durch die nachfolgenden Beispiele weiter verdeutlicht.

**Beispiele 1-9 und Vergleichsbeispiele 1-4**

Acrolein wird unter den in der Tabelle angegebenen Bedingungen zu 3-Hydroxypropionaldehyd (HPA) hydratisiert. Durch Zugabe von Acrolein zu Pufferlösungen der angegebenen Zusammensetzung wird die angegebene Acrolein-Startkonzentration eingestellt. Das erhaltene Reaktionsgemisch wird während der angegebenen Reaktionsdauer bei der angegebenen Reaktionstemperatur gerührt. Anschließend werden der Umsatz an Acrolein und die Selektivität bezüglich HPA gaschromatographisch bestimmt. Der Tabelle sind die Bedingungen und Ergebnisse der Beispiele 1 bis 9 und der Vergleichsbeispiele VB1 bis VB4 zu entnehmen.

T a b e l l e

| Beispiel | Katalysatorlösung Wasser (g) | Säure/ (g) | Base/ (g) | pH | Hydratisierung von Acrolein Acrolein-Startkonz. (Gew.-%) | Temp. ($^{o}C$) | Dauer (h) | Umsatz (%) | Selektivität (%) |
|---|---|---|---|---|---|---|---|---|---|
| VB1 | 180 | PrS/8 | - | 2,5 | 17 | 80 | 2 | 24,5 | 52,1 |
| 1 | 180 | PrS/8 | TEA/0,85 | 4,0 | 17 | 70 | 2 | 44,1 | 85,8 |
| VB2 | 180 | PrS/0,01 | - | 4,0 | 17 | 70 | 2 | 2,0 | |
| 2 | 180 | EsS/8 | TEA/0,38 | 3,0 | 17,2 | 70 | 2 | 32,9 | 84,8 |
| 3 | 180 | EsS/8 | TEA/0,73 | 3,3 | 17,1 | 70 | 2 | 40,1 | 86,1 |
| 4 | 180 | EsS/8 | TEA/2,22 | 3,9 | 17,3 | 70 | 2 | 49,9 | 84,3 |
| 5 | 180 | EsS/8 | NL/24,4 | 4,5 | 17,7 | 70 | 2 | 57,1 | 83,2 |
| VB3 | 180 | EsS/8 | - | 2,4 | 17,0 | 70 | 2 | 16,0 | 60,2 |
| 6 | 180 | iBuS/8 | TEA/1,3 | 4,0 | 16,8 | 70 | 2 | 50,4 | 86,9 |
| 7 | 233 | AcS/1,8 | TEA/0,29 | 3,0 | 15,9 | 90 | 3 | 53,8 | 77,0 |
| 8 | 180 | $H_3PO_4$/9,4 | TEA/8,4 | 4,1 | 16,6 | 70 | 2 | 60,7 | 80,3 |
| 9 | 180 | $H_3PO_4$/9,4 | DBA/10,6 | 4,0 | 16,8 | 60 | 2 | 58,0 | 47,9 |
| VB4 | 180 | $H_3PO_4$/$2 \times 10^{-5}$ | - | 4,0 | 16,8 | 70 | 2 | < 1 | |

VB = Vergleichsbeispiel (Stand der Technik)

EsS = Essigsäure
PrS = Propionsäure
iBuS = Isobuttersäure
AcS = Acrylsäure

TEA = Triethylamin
DBA = Dibutylamin
NL = Natronlauge 10 %ig

**Beispiel 10**

Gesamtverfahren:

Acrolein $\xrightarrow{H_2O}$ HPA $\xrightarrow{H_2}$ 1,3-Propandiol einschließlich Rückgewinnung des Katalysators $RCOOH/NR_3$.

Es wurden 1000 g $H_2O$, 45 g Propionsäure (PrS) und 5 g Triethylamin (TEA) gemischt, so daß sich ein pH-Wert von etwa 4 einstellte. Zu dieser Lösung gab man 210 g Acrolein. Das Acrolein wurde dann in einem Reaktionsrohr bei einem LHSV-Wert von 0,5 $h^{-1}$ bei 70 °C umgesetzt.

Anschließend wurde das nicht umgesetzte Acrolein bei vermindertem Druck (350 mbar) von der wäßrigen HPA-Lösung abgetrennt. Der Acroleinumsatz betrug 45 %, die Selektivität bezüglich HPA 85 %. Die HPA-Lösung (HPA-Konzentration = 8,8 Gew.-%) wurde in einem Hydrierautoklaven mit Begasungsrührer hydriert. Der $H_2$-Druck betrug 135 bar, die Reaktionstemperatur 60 °C; als Katalysator wurden 5 g Raney-Nickel eingesetzt. Die Ausbeute an 1,3-Propandiol (PD), bezogen auf eingesetztes HPA, betrug 99,8 %.

Nach der Hydrierung wurden in einer Destillationskolonne bei 50 mbar Wasser und Katalysator abdestilliert. In dem wäßrigen Destillat wurden nahezu 100 % des eingesetzten TEA und 97 % der eingesetzten Propionsäure wiedergefunden.

Das im Sumpf der Destillationskolonne zurückbleibende PD wurde destillativ gereinigt. Die Gesamtausbeute an 1,3-Propandiol, bezogen auf eingesetztes Acrolein, betrug 83 %.

**Patentansprüche**

**1.** Verfahren zur Herstellung von 3-Hydroxyalkanalen mit 3 bis 12 C-Atomen durch Hydratisierung der zugrundeliegenden 2-Alkenale mit Wasser in homogener Phase in Gegenwart eines sauren Katalysatores bei einer Reaktionstemperatur von 20 °C bis 120 °C, einem Druck von 1 bar bis 20 bar und einer Anfangskonzentration des 2-Alkenals im Reaktionsgemisch von 3 bis 30 Gew.-%,
dadurch gekennzeichnet,
daß man als Katalysator einen gelösten Säure-Base-Puffer auf der Basis (a) einer einwertigen Carbonsäure und eines löslichen Salzes dieser Säure oder (b) einer mehrwertigen Carbonsäure oder Phosphorsäure und eines Salzes aus diesen Säuren mit tertiären Aminen oder N-Heteroaromaten verwendet, der im Reaktionsgemisch zu einem pH-Wert von 2 bis 5 führt und dessen Säurekomponente und korrespondierende Base im Reaktionsgemisch in einer Menge von insgesamt 0,5 bis 40 Gew.-% anwesend sind.

**2.** Verfahren nach Anspruch 1 oder 2,
dadurch gekennzeichnet,
daß man einen Säure-Base-Puffer auf der Basis einer einwertigen Carbonsäure mit einem Siedepunkt unter Normaldruck von unter 200 °C, vorzugsweise unter 160 °C, und eines Salzes derselben mit einem tertiären Amin oder N-Heteroaromat verwendet, wobei das tertiäre Amin oder N-Heteroaromat einen Siedepunkt unter Normaldruck von unter 200 °C, vorzugsweise unter 160 °C, aufweist.

**3.** Verfahren nach Anspruch 1,
dadurch gekennzeichnet,
daß man den pH-Wert im Reaktionsgemisch mit dem Säure-Base-Puffer auf 3 bis 4,5 einstellt.

**4.** Verfahren nach einem oder mehreren der Ansprüche 1 bis 3,
dadurch gekennzeichnet,
daß man als Säurekomponente des Puffers Essigsäure, Propionsäure oder Acrylsäure verwendet.

**5.** Verfahren nach einem oder mehreren der Ansprüche 1 bis 4,
dadurch gekennzeichnet,

daß man zur Herstellung des Säure-Base-Puffers eine Säure, vorzugsweise eine niedere Carbonsäure, und ein Amin aus der Reihe Trimethylamin, Triethylamin, Tripropylamin oder Pyridin verwendet.

6. Verfahren nach einem oder mehreren der Ansprüche 1 bis 5,
dadurch gekennzeichnet,
daß man die Hydratisierung bei 50 bis 90 °C durchführt.

7. Verfahren nach einem oder mehreren der Ansprüche 1 bis 6,
dadurch gekennzeichnet,
daß man die Anfangskonzentration des 2-Alkenals auf 6 bis 20 Gew.-%, vorzugsweise 10 bis 18 Gew.-%, bezogen auf das Reaktionsgemisch, einstellt.

8. Verfahren nach einem oder mehreren der Ansprüche 1 bis 7,
dadurch gekennzeichnet,
daß im Reaktionsgemisch 0,5 bis 10 Gew.-%, vorzugsweise 0,5 bis 5 Gew.-%, bezogen auf das Reaktionsgemisch, der Säurekomponente und seiner korrespondierenden Base anwesend sind.

9. Verfahren nach einem oder mehreren der Ansprüche 1 bis 8,
dadurch gekennzeichnet,
daß man als 2-Alkenal Acrolein einsetzt.

10. Verwendung des nach Anspruch 9 in Verbindung mit einem oder mehreren der Ansprüche 2 bis 8 nach Beendigung der Hydratisierung erhaltenen und von nicht umgesetztem Acrolein befreiten Reaktionsgemischs zur Herstellung von 1,3-Propandiol.

## Claims

1. Process for the production of 3-hydroxyalkanals having 3 to 12 carbon atoms by hydration of the corresponding 2-alkenals with water in a homogeneous phase in the presence of an acid catalyst at a reaction temperature of 20 °C to 120 °C, a pressure of 1 bar to 20 bar and an initial concentration of the 2-alkenal in the reaction mixture of 3 to 30 wt.%,
characterised in that
the catalyst used is a dissolved acid-base buffer based on (a) a monobasic carboxylic acid and a soluble salt of this acid or (b) a polybasic carboxylic acid or phosphoric acid and a salt of these acids with tertiary amines or N-heteroaromatic compounds, resulting in a pH of 2 to 5 in the reaction mixture, the acid component and corresponding base being present in the reaction mixture in a total quantity of 0.5 to 40 wt.%.

2. Process according to claim 1,
characterised in that
an acid-base buffer based on a monobasic carboxylic acid having a boiling point at standard pressure of below 200 °C, preferably of below 160 °C, and a salt thereof with a tertiary amine or an N-heteroaromatic compound is used, wherein the tertiary amine or N-heteroaromatic compound has a boiling point at standard pressure of below 200 °C, preferably of below 160 °C.

3. Process according to claim 1,
characterised in that
the pH in the reaction mixture is adjusted to 3 to 4.5 with the acid-base buffer.

4. Process according to one or more of claims 1 to 3,
characterised in that
the acid component of the buffer used is acetic acid, propionic acid or acrylic acid.

5. Process according to one or more of claims 1 to 4,
characterised in that
an acid, preferably a lower carboxylic acid, and an amine selected from trimethylamine, triethylamine, tripropylamine or pyridine are used for production of the acid-base buffer.

6. Process according to one or more of claims 1 to 5, characterised in that hydration is performed at 50 to 90 ° C.

7. Process according to one or more of claims 1 to 6, characterised in that the initial concentration of the 2-alkenal is adjusted to 6 to 20 wt.%, preferably to 10 to 18 wt.%, relative to the reaction mixture.

8. Process according to one or more of claims 1 to 7, characterised in that 0.5 to 10 wt.%, preferably 0.5 to 5 wt.%, relative to the reaction mixture, of the acid component and its corresponding base are present in the reaction mixture.

9. Process according to one or more of claims 1 to 8, characterised in that the 2-alkenal used is acrolein.

10. Use of the reaction mixture obtained according to claim 9 in combination with one or more of claims 2 to 8 on termination of hydration, from which unreacted acrolein has been removed, for the production of 1,3-propanediol.

**Revendications**

1. Procédé de préparation de 3-hydroxyalcanales ayant de 3 à 12 atomes de C par hydratation des 2-alcénals sous-jacents avec de l'eau en phase homogène en présence d'un catalyseur acide à une température de réaction de 20 ° C à 120 ° C, une pression de 1 bar à 20 bars et une concentration initiale du 2-alcénal dans le mélange réactionnel de 3 à 30 % en poids, caractérisé en ce qu'on utilise comme catalyseur un tampon acide-base à base de (a) un acide carboxylique monovalent et un sel soluble de cet acide ou de (b) un acide carboxylique plurivalent ou de l'acide phosphorique et un sel de ces acides avec des amines tertiaires ou des hétéro-aromates de N, tampon qui dans le mélange réactionnel conduit à une valeur de pH de 2 à 5 et dont le composant acide et la base correspondante sont présents dans le mélange réactionnel en une quantité totale de 0,5 à 40 % en poids.

2. Procédé selon les revendications 1 ou 2, caractérisé en ce qu'on utilise un tampon acide-base à base d'un acide carboxylique monovalent ayant un point d'ébullition à pression normale inférieur à 200 ° C, de préférence inférieur à 160 ° C, et un sel de celui-ci même avec une amine tertiaire ou un hétéro-aromate de N, l'amine tertiaire ou hétéro-aromate de N ayant un point d'ébullition à pression normale inférieur à 200 ° C, de préférence inférieur à 160 ° C.

3. Procédé selon la revendication 1, caractérisé en ce qu'on ajuste la valeur du pH dans le mélange réactionnel avec le tampon acide-base de 3 à 4,5.

4. Procédé selon une ou plusieurs des revendications 1 à 3, caractérisé en ce qu'on utilise comme composant acide du tampon de l'acide acétique, de l'acide propionique ou de l'acide acrylique.

5. Procédé selon une ou plusieurs des revendications 1 à 4, caractérisé en ce qu'on utilise pour la préparation du tampon acide-base un acide, de préférence un acide carboxylique inférieur, et une amine de la série triméthylamine, triéthylamine, tripropylamine ou pyridine.

6. Procédé selon une ou plusieurs des revendications 1 à 5, caractérisé en ce qu'on exécute l'hydratation de 50 à 90 ° C.

7. Procédé selon une ou plusieurs des revendications 1 à 6, caractérisé en ce qu'on ajuste la concentration initiale du 2-alcénal de 6 à 20 % en poids, de préférence de 10 à 18 % en poids, rapportée au mélange réactionnel.

8. Procédé selon une ou plusieurs des revendications 1 à 7, caractérisé en ce que dans le mélange réactionnel sont présents de 0,5 à 10 % en poids de préférence de 0,5 à 5 % en poids, par rapport au mélange réactionnel du composant acide et de la base correspondante.

9. Procédé selon une ou plusieurs des revendications 1 à 8, caractérisé en ce qu'on met en oeuvre en tant que 2-alcénal de l'acroléine.

10. Utilisation du mélange réactionnel obtenu selon la revendication 9 en liaison avec une ou plusieurs des revendications 2 à 8 après la fin de l'hydratation et après élimination de l'acroléine non transformée, pour la préparation de 1,3-propandiol.